# EUROPEAN PATENT APPLICATION

(11) **EP 2 036 520 A1**
(43) Date of publication of application: **18.03.2009**
(21) Application number: 06767537.1
(22) Date of filing: 28.06.2006
(51) Int. Cl.: A61F 5/02, A61H 39/04

(54) **WAIST BELT**

(71) Applicant: Sea Shell Co., Ltd., Osaka-shi, Osaka 543-0001 (JP); Kawahara, Takemasa, Tennoji-ku Osaka-shi Osaka 5430001 (JP)
(72) Inventor: KAWAHARA, Takemasa, Osaka-shi Osaka 543-0001 (JP)
(74) Representative: Benedum, Ulrich Max
(86) International application number: PCT/JP2006/312920
(87) International publication number: WO 2008/001440

(57) **Abstract**

To provide a waist belt having an improved constitution by considering the structure of members that compress the posterior superior and inferior iliac spines and the direction of the compression so as to stabilize and correct the sacroiliac joint based on the pelvic structure. A waist belt having concave parts at the parts to be in contact with the posterior superior and inferior iliac spines (50) of the iliac bone in the back face of the belt body (2), wherein cup-shaped iliac bone pads (5, 6) having projections (7, 8) are formed at the bottom of the concave parts and the posterior superior and inferior iliac spines (50) are compressed downward by the projections (7, 8). At the parts having these cup-shaped iliac bone pads (5, 6) in the front face side of the belt body (2), X-shaped auxiliary belts (9, 10) are provided for compressing the cup-shaped iliac bone pads (5, 6) at the intersecting point of the X-shape to thereby transfer the compression pressure.

## Description

### FIELD OF THE INVENTION

The present invention relates to a waist belt, and particularly to a waist belt that not only exhibits a function for compressing, from directions within a specific range, the iliac bones that constitute the pelvis, but can stabilize the sacroiliac joint.

### BACKGROUND ART

When having gained the ability the walk on two legs humans have a S-shaped spine to support a heavy cranium and a lumbar spine to support the entire weight of the upper body. Nonetheless, the transition to walking on two legs from walking on all fours has led to a tendency that the pelvis is tilted forward when in an erect standing posture. Loads tend to be placed on the lumbar spine because of a skeletal structure which not only exposes everyone to the risk of developing back pain but also readily causes the body axis to fall out of alignment.

A variety of waist belts have been proposed in order to alleviate and reduce the back pain that inevitably occurs from walking on two legs, or in order to correct distortion that arises in the pelvis. Examples of waist belts include for example corsets designed to be applied on the lumbar region and which entire structure is formed of a plastic or other material having a certain degree of flexibility; supporters designed so that the lumbar region will be wrapped with a wide fabric composed of an elastic material, the ends thereof being fastened together by hook-and-loop fasteners; and other similar products (JP 6-78943, JP 2004-277936; JP 2005-192677).

In addition to corsets and supporters that are mainly intended to alleviate and stabilize back pain, a variety of types have been proposed in which therapeutic pressure projections, compression plates, or other components are disposed on regions of the belt that come into contact with the lumbar region in order to exhibit a therapeutic pressure effect for promoting the circulation of the blood and a corrective effect on distortions of the pelvis (JP-UM 60-168811; JP 11-253524; JP 2002-345866)

### DISCLOSURE OF THE INVENTION

### [Problems to be solved by the invention]

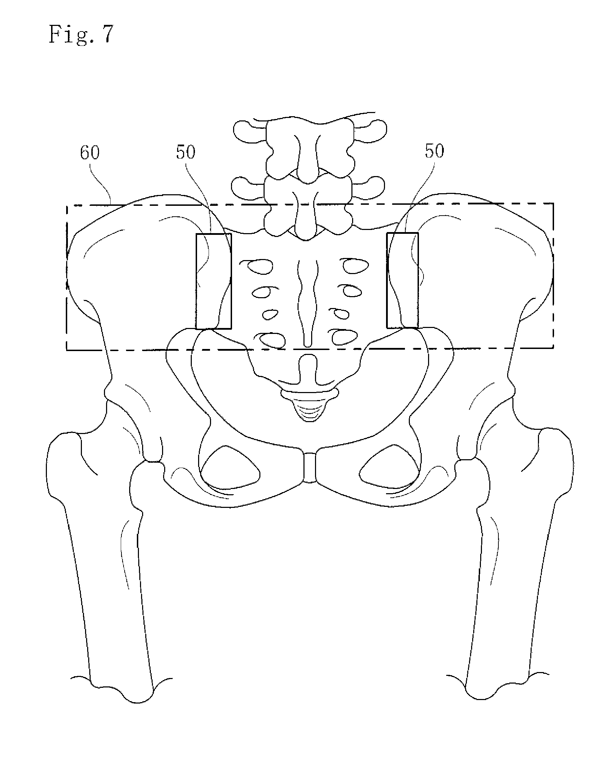

As shown in FIG. 7, a waist belt 60 cited in the above patent documents is wrapped around the entire pelvis in the so-called sacroiliac joint region, where the two iliac bones are joined to the one sacral bone, thereby creating a structure in which wearing the belt applies pressure to the entire pelvis. The broken line in the drawing shows the belt 60. The therapeutic pressure projections and pressure plates act simply to compress the sacral bone region, and are merely disposed so as to promote blood circulation and exhibit a corrective function.

Wearing the belt causes the iliac bones to be compressed from the rear of the lumbar region, or from the front left and right of the lumbar region, so that the width between the iliac bones is adjusted and back pain is remedied. However, the simple compression function alone does not deliver a sufficient force in the stabilizing direction in terms of the structure of the sacroiliac joint. Specifically, in order to stabilize the sacroiliac joint, a design is needed so that pressure will be applied in a direction that effectively corrects the forward tilt of the pelvis, and so that the applied compressive force will be no more than an appropriate force that does not impart excessive load to the wearer. However, such points have not been taken into consideration in the abovementioned conventional waist belts. In particular, the action shown in FIG. 7 on the posterior superior and inferior iliac spines 50 has been problematic.

With the foregoing in view, it is an object of the present invention to provide a waist belt having a more suitable construction, particularly in consideration of the structure of the compressive members for the posterior superior and inferior iliac spines and also the direction in which that pressure is applied, in order to achieve correction and stabilization of the sacroiliac joint while taking the pelvic structure into account.

### [Measures for solving the above-mentioned problems]

The present invention is constructed as described below in order to solve the above-mentioned problems. Specifically, the waist belt according to claim 1 is a waist belt formed so as to be removably attached to the lumbar region, the iliac bones being enclosed by an elastic belt body, the waist belt characterized in comprising: a cup-shaped iliac bone pad in a region to be in contact with the posterior superior and inferior iliac spines of the iliac bones on a back face of the belt body, the cup-shaped iliac bone pad having a shape of a concavity, a projection being provided on an interior bottom of the concavity, and the projection pushing and compressing the posterior superior and inferior iliac spines inward and downward; and an x-shaped auxiliary belt on a front face of the belt body in the region to which the cup-shaped iliac bone pad is provided, the auxiliary belt exhibiting an elastic force so as to compress and transmit a pressing force to the cup-shaped iliac bone pad at a point of intersection of the x-shape.

The waist belt according to claim 2 is characterized in that the projection of the cup-shaped iliac bone pad receives the elastic force of the auxiliary belt, and a direction in which the posterior superior and inferior iliac spines are compressed inward and downward is set for a vertical direction at a maximum range of from 10° to 120° from a vertical reference line, the vertical reference line being derived from a line drawn from the acromion to the greater trochanter of the femur and moved in parallel over to the posterior superior and inferior iliac spines, and is set for a horizontal direction at a maximum of from 10° to 120° from a horizontal reference line, the horizontal reference line being derived from a line from the posterior superior and inferior iliac spines that intersects the vertical reference line at a right angle.

### [Effect of the Invention]

According to the waist belt of claim 1, cup-shaped iliac bone pads are provided to a region that comes into contact with the posterior superior and inferior iliac spines of the iliac bones, and as long as the belt body is put on and then worn so that the cup-shaped iliac bone pads are compressed by the auxiliary belts, the projections provided to the cup-shaped iliac bone pads act so as to push the posterior superior and inferior iliac spines in an inward and downward direction; therefore, the sacroiliac joint is corrected to the inherently proper position and the lumbar region is stabilized.

According to the waist belt of claim 2, the angle to which the projections push the posterior superior and inferior iliac spines inward and downward is set to within a suitable range considering individual differences in the pelvic structures of men and women, whereby the effectiveness of the waist belt can be more clearly exhibited.

### BEST MODE FOR CARRYING OUT THE INVENTION

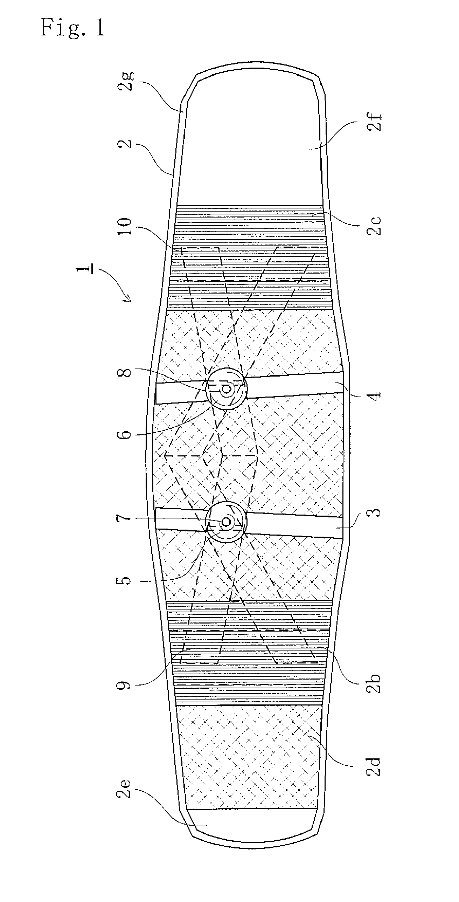

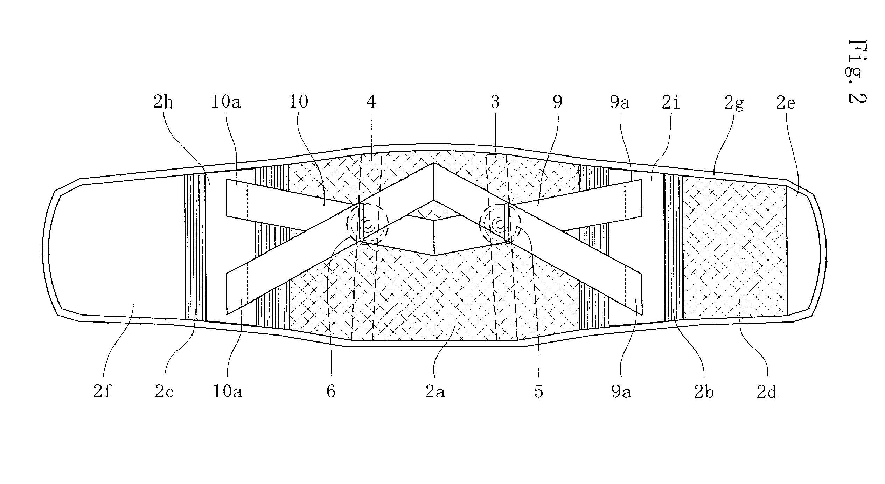

An embodiment of the present invention is described in detail herein below with reference to FIGS, 1 to 6. FIG. 1 is a plan view of a waist belt 1 according to the present invention, showing the side that faces the body (the underlining). FIG. 2 is a plan view of the waist belt 1, showing the front face side, which does not touch the stomach or back (the top fabric). The waist belt 1 comprises a belt body 2 composed of an elastic material, a pair of cup-shaped iliac bone pads 5, 6 to which projections 7, 8 are provided, and x-shaped auxiliary belts 9, 10.

The belt body 2 comprises a central elastic mesh part 2a mainly made of a polyester material, elastic parts 2b, 2c formed of a rubber material being more elastic than the mesh part and situated on either side of the elastic mesh part 2a, a mesh part 2d that connects to the far side of the elastic part 2b, a hook-and-loop fastener 2e that connects to the far side of the mesh part 2d and has a hook portion on the reverse side, and a hook-and-loop fastener 2f that connects to the far side of the elastic part 2c and has a loop pile. The periphery of these parts is stitched using a tape cloth 2g, and the borders of each of the above-described parts are also stitched.

As shown in FIG. 1, either side of the belt body 2 along a central axis line is stitched with a base fabric 3, 4, within which plate-shaped members have been sewn. The cup-shaped iliac bone pads 5, 6 are rotatably provided to the regions of the base fabric 3, 4 that come into contact with the posterior superior and inferior iliac spines of the iliac bones when the belt body 2 is placed around the waist.

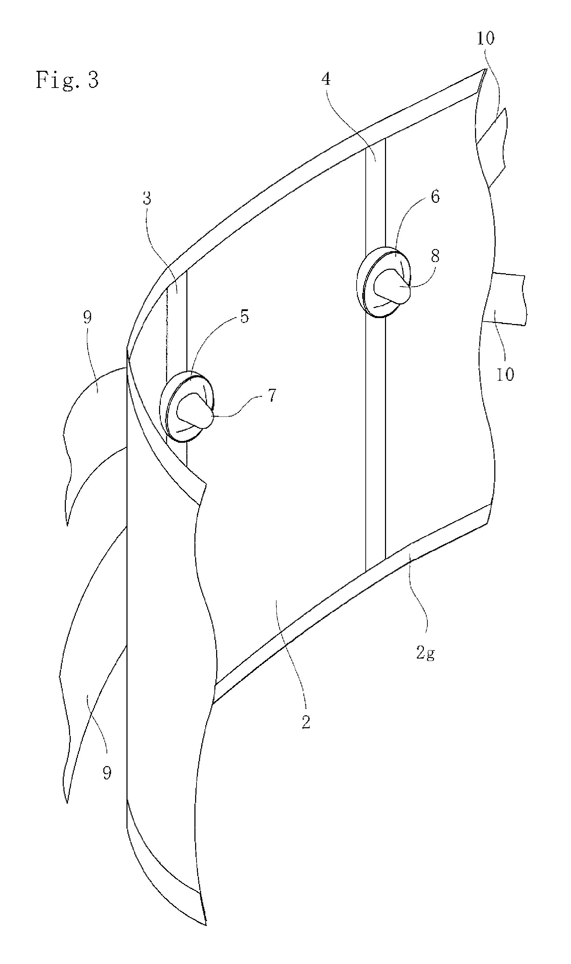

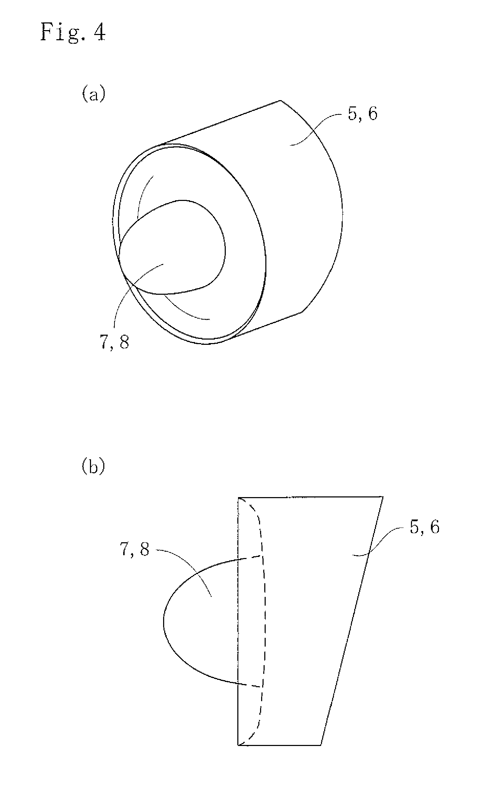

As shown in FIG. 3, which is a perspective view of the parts of the present embodiment, the projections 7, 8 are mounted inside and on the bottom of the cup-shaped concave surface of each of the cup-shaped iliac bone pads 5, 6. As shown in FIGS. 4a, 4b, which are respectively an exterior perspective view and a side view, the ends of the cup-shaped iliac bone pads 5, 6 that adjoin to the belt body 2 are formed as inclined surfaces. By setting the angle of the inclined surfaces and rotating and adjusting the direction in which the cup-shaped iliac bone pads 5, 6 attach to the belt body 2, the direction in which the posterior superior and inferior iliac spines (described hereinafter) will be compressed inward and downward can be determined.

As shown in FIG. 2, x-shaped auxiliary belts 9, 10 are provided to the reverse side of the region in which the cup-shaped iliac bone pads 5, 6 are disposed; i.e., the surface of the waist belt 1 that does not touch the stomach and back. The auxiliary belts 9, 10 are formed from an elastic material that exhibits elasticity in order to compress the cup-shaped iliac bone pads 5, 6 at the point of intersection of the x-shape.

One end of each of the auxiliary belts 9, 10 is stitched along the central axis line of the belt body 2, the point of intersection of the x-shape is stitched on the reverse side of the base fabric 3, 4 in which are sewn plate-shaped members to which the cup-shaped iliac bone pads 5, 6 are attached, and hook-and-loop fasteners 9a, 10a having a hook portion are formed on the other ends of the auxiliary belts 9, 10. Hook-and-loop fasteners 2h, 2i, which have a loop pile with which the hook-and-loop fasteners 9a, 10a formed on the ends of the auxiliary belts 9, 10 lock and adhere, are stitched on the front face of the elastic parts 2b, 2c that are associated with the belt body 2 and are formed of an elastic rubber material.

When the belt body 2 is wrapped around the circumference of the waist while resisting the elasticity of the elastic parts 2b, 2c, and is attached using the hook-and-loop fasteners 2e, 2f to be worn, the perimeter of the circular shape of the abovementioned cup-shaped iliac bone pads 5, 6 comes into contact with left and right posterior superior and inferior iliac spines 50 of the pelvis in an encircling manner. The projections 7, 8 provided to the bottom of the interior of the concave surface of the cup-shaped iliac bone pads 5, 6 are disposed so as to compress the posterior superior and inferior iliac spines 50. The pads 5, 6 are made of an elastic and highly adhesive material, in contrast to the projections 7, 8, so that the pads 5, 6 will not be displaced when being made to come into contact with and apply pressure to the posterior superior and inferior iliac spines 50 as a result of being formed in the shape of a cup. A material having a high degree of hardness is used for the projections 7, 8 in order to enhance the effect of the compression.

With the belt body 2 in a fitted state, when the hook-and-loop fasteners 9a, 10a of the x-shaped auxiliary belts 9, 10 lock and adhere to the hook-and-loop fasteners 2h, 2i of the belt body 2 while the end parts [on which the fasteners 9a, 10a] are formed are pulled outward, the points of intersection of the 'x' shapes of the auxiliary belts 9, 10 compress further and apply pressure to the cup-shaped iliac bone pads 5, 6 provided to the reverse side. When pressure is applied to the cup-shaped iliac bone pads 5, 6 from the auxiliary belts, as described above, the projections 7, 8 act so as to push the posterior superior and inferior iliac spines 50 in an inward and downward direction, which is set according to the angle setting of the inclined surfaces of the cup-shaped iliac bone pads 5, 6, and the direction in which the cup-shaped iliac bone pads 5, 6 are attached to the belt body 2 through rotational adjustment.

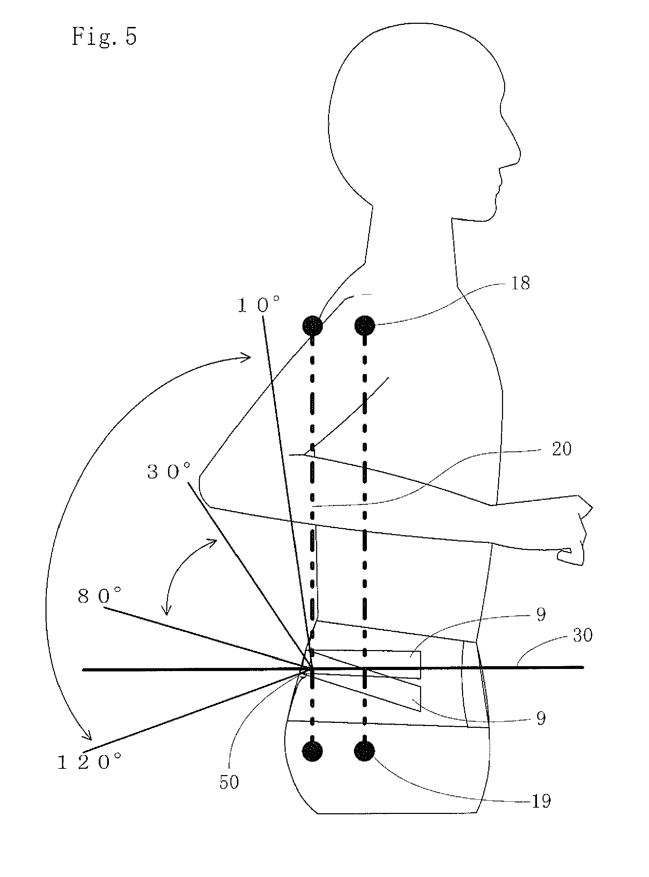

Specifically, as shown in FIG. 5, the inward and downward facing direction is set at a maximum range of 10° to 120° in relation to the vertical direction, the point of reference being the intersection of a vertical reference line 20 and a horizontal line 30 drawn forward and rearward through the posterior superior and inferior iliac spines 50 at a right angle to the axis of the body. The vertical reference line 20 is a line derived from a line that is drawn from the caroming 18 to the greater trochanter 19 of the femur of the wearer and is moved to the posterior superior and inferior iliac spines 50 in parallel.

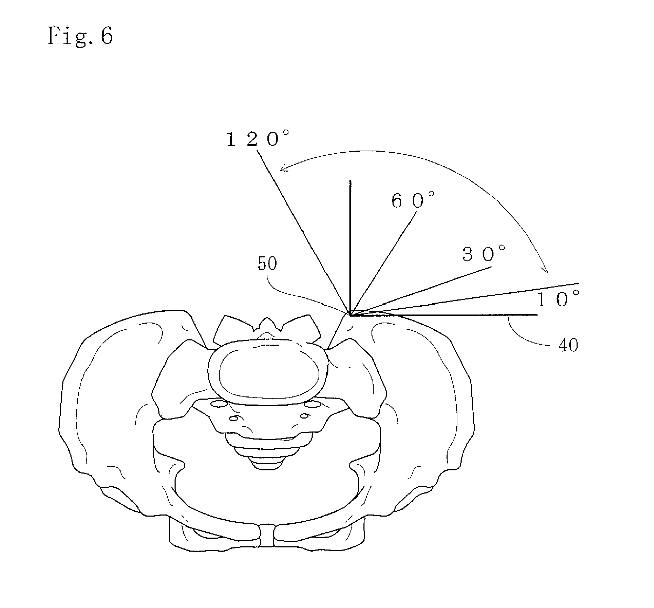

As shown in FIG. 6, the direction is set to a horizontal range that is a maximum of 10° to 120° with reference to the point of intersection between the vertical reference line 20 and a horizontal line 40 that intersects the posterior superior and inferior iliac spines 50 at right angles to the left and the right. FIG. 5 shows an illustration of the left side posterior superior and inferior iliac spines 50; for the right side posterior superior and inferior iliac spines 50 the reference directions are reversed.

Having the posterior superior and inferior iliac spines 50 pushed in the inward and downward direction determined for both elements causes the entire sacroiliac joint as a whole to be corrected and made to assume an inherently proper position, whereby the lumbar region is stabilized. In conventional waist belts, no consideration has been given to the corrective effects brought about by the direction in which pressure is applied. The present invention is characterized in that the direction of pushing toward the posterior superior and inferior iliac spines 50 is set to be the inward and downward direction, and is set to the abovementioned range as a range in which the action occurs to a certain degree of effectiveness in consideration of the structure of the pelvis and the individual differences between men and women. However, based on the standard described above, an angle from 30° to 80° from the vertical direction, and from 30° to 80° from the horizontal direction, is recommended as an optimal angle for greater stability, and orienting the settings to these directions enables the corrective effects of the waist belt to be demonstrated to an increased degree of reliability.

### INDUSTRIAL APPLICABILITY

In the above-described embodiment, the cup-shaped iliac bone pads 5, 6 are irremovably provided; however, a removable construction may be adopted, and a variety of pads may be prepared by varying the angle of the inclined surfaces of the cup-shaped iliac bone pads 5, 6 and having different ranges of set angles at which the projections 7, 8 push toward the posterior superior and inferior iliac spines 50. This arrangement will provide interchangeability, thereby enabling the gender and physical build of the wearer to be taken into account, and a broader range of application to be realized. Also, in the embodiment, the direction in which the cup-shaped iliac bone pads 5, 6 are attached to the installation sites is configured so that the cup-shaped iliac bone pads 5, 6 can be rotated and the angle at which the projections 7, 8 push toward the posterior superior and inferior iliac spines 50 can be adjusted. In an alternative arrangement, the sites in which the cup-shaped iliac bone pads 5, 6 are attached can be moved, and the spacing over which the pads 5, 6 are situated or the relative positioning of the sites can also be unrestrictedly adjusted, so that it is possible to demonstrate an effect of pushing toward the posterior superior and inferior iliac spines 50 at pressure points that are more suitable for the wearer.

The cup-shaped iliac bone pads 5, 6 are made of an elastic and highly adhesive material, in contrast to the projections 7, 8, so that the pads 5, 6 will not be displaced when being made to come into contact with and apply pressure to the posterior superior and inferior iliac spines 50 as a result of being formed in the shape of a cup. A material having a high degree of hardness is used for the projections 7, 8 in order to enhance the effect of the compression. However, there are also cases in which improving the adhesion characteristics of the projections 7, 8 is more appropriate for the user, and the configuration of the materials may be completely reversed.

### BRIEF DESCRIPTION OF THE DRAWINGS

FIG. 1 is a plan view seen from the interior, showing the construction of a waist belt according to an embodiment of the present invention;
FIG. 2 is a plan view seen from the exterior, showing the construction of a waist belt according to an embodiment of the present invention;
FIG. 3 is a partial perspective view showing the structure of the essential parts of the embodiment;
FIG. 4a is a perspective view showing the structure of the cup-shaped iliac bone pads;
FIG. 4b is a side view showing the structure of the cup-shaped iliac bone pads;
FIG. 5 shows a case in which the projections apply pressure in the vertical direction toward the posterior superior and inferior iliac spines of the embodiment;
FIG. 6 shows a case in which the projections apply pressure in the horizontal direction toward the posterior superior and inferior iliac spines of the embodiment; and
FIG. 7 is a drawing showing a conventional waist belt in a state of use.

### REFERENCE NUMERALS

- 1: WAIST BELT
- 2: BELT BODY
- 5, 6: CUP-SHAPED ILIAC BONE PAD
- 7, 8: PROJECTION
- 9, 10: AUXILIARY BELT
- 50: POSTERIOR SUPERIOR AND INFERIOR ILIAC SPINES

## Claims

1. A waist belt formed so as to be removably attached to the lumbar region, the iliac bones being enclosed by an elastic belt body, the waist belt **characterized in** comprising:
a cup-shaped iliac bone pad in a region to be in contact with the posterior superior and inferior iliac spines of the iliac bones on a back face of the belt body, the cup-shaped iliac bone pad having a shape of a concavity, a projection being provided on an interior bottom of the concavity, and the projection pushing and compressing the posterior superior and inferior iliac spines inward and downward; and
an x-shaped auxiliary belt on a front face of the belt body in the region to which the cup-shaped iliac bone pad is provided, the auxiliary belt exhibiting an elastic force so as to compress and transmit a pressing force to the cup-shaped iliac bone pad at a point of intersection of the x-shape.

2. The waist belt according to claim 1, **characterized in that** the projection of the cup-shaped iliac bone pad receives the elastic force of the auxiliary belt, and a direction in which the posterior superior and inferior iliac spines are compressed inward and downward is set for a vertical direction at a maximum range of from 10° to 120° from a vertical reference line, the vertical reference line being derived from a line drawn from the acromion to the greater trochanter of the femur and moved in parallel over to the posterior superior and inferior iliac spines, and is set for a horizontal direction at a maximum of from 10° to 120° from a horizontal reference line, the horizontal reference line being derived from a line from the posterior superior and inferior iliac spines that intersects the vertical reference line at a right angle.
